# FASCICULE DE BREVET EUROPEEN

(11) **EP 0 935 974 B1**
(45) Date de publication et mention de la délivrance du brevet: **08.10.2003**
(21) Numéro de dépôt: 99400220.2
(22) Date de dépôt: 02.02.1999
(51) Int. Cl.: A61M 25/00, A61M 29/00

(54) **Dispositif télescopique pour la dilatation d'un canal corporel**
Teleskopische Vorrichtung zur Erweiterung eines Körperkanals
Telescopic device to dilate a body vessel

(30) Priorité: 11.02.1998 FR 9801619
(43) Date de publication de la demande: 18.08.1999
(73) Titulaire: PORGES, 92350 Le Plessis-Robinson (FR)
(72) Inventeur: Ismael, Bernard, 75019 Paris (FR)
(74) Mandataire: Bonnetat, Christian

(56) Documents cités:
- EP-A- 0 334 116
- DE-A- 3 936 811
- FR-A- 2 658 082
- US-A- 3 811 449

## Description

La présente invention concerne un dispositif télescopique pour la dilatation d'un canal corporel.

On sait que, par exemple pour le passage d'une sonde d'exploration ou de tout autre instrument, il est usuel de dilater un canal corporel, tel que l'urètre, le canal cystique, etc ... Par exemple, pour pouvoir explorer les voies biliaires à l'aide d'un cholédoscope passant par le canal cystique, il est la plupart du temps nécessaire de dilater ce canal avant l'introduction dudit cholédoscope.

Pour pouvoir effectuer de telles dilatations de canaux corporels, on connaît déjà plusieurs types de dispositifs de dilatation. Il s'agit essentiellement de :
- sondes à ballonnet gonflable, dans lesquelles la pression de gonflage est contrôlée ;
- jeux de bougies de dilatation individuelles de diamètres différents, lesdites bougies étant introduites successivement, par ordre de diamètre croissant, dans le canal à dilater ;
- jeux de tubes souples individuels de diamètres différents, enfilés successivement par ordre de diamètre croissant les uns sur les autres en étant guidés sur un fil-guide préalablement mis en place dans ledit canal.

Ce dernier type de dispositif de dilatation présente l'inconvénient d'une introduction successive d'éléments tubulaires individuels par ordre de diamètre croissant, ce qui rend cette introduction longue et délicate. De plus, il ne permet pas d'obtenir des dispositifs de dilatation de petit diamètre, susceptibles d'être utilisés dans des petits canaux corporels, comme par exemple le canal cystique.

Par ailleurs, par exemple par les documents FR-A-2 658 082 et DE-A-39 36 811, on connaît déjà un dispositif pour la dilatation d'un canal corporel comportant des tubes de diamètres différents susceptibles de coulisser l'un dans l'autre, dispositif dans lequel :
- lesdits tubes sont emboîtés l'un dans l'autre pour former une unité télescopique de dilatation dans laquelle :
   . la longueur d'un tube est d'autant plus grande que le diamètre de celui-ci est plus petit ;
   . l'extrémité distale d'un tube est en saillie par rapport à l'extrémité distale du tube de diamètre immédiatement supérieur ;
   . l'extrémité proximale d'un tube est en saillie par rapport à l'extrémité proximale du tube de diamètre immédiatement supérieur ; et
   . l'extrémité proximale de chaque tube est pourvue d'une butée proximale de coulissement ; et
- ladite unité télescopique peut prendre au moins :
   . une position d'introduction dans le canal naturel, pour laquelle :
      * l'extrémité proximale de ladite unité télescopique présente, au-delà de l'extrémité proximale du tube de plus grand diamètre, une extension longitudinale minimale, avec lesdites butées proximales de coulissement en contact ; et
      * l'extrémité distale de ladite unité télescopique présente, au-delà de l'extrémité distale du tube de plus grand diamètre, une extension longitudinale maximale ; et
   . une position de dilatation maximale dudit canal naturel, pour laquelle :
      * l'extrémité proximale de ladite unité télescopique présente, au-delà de l'extrémité proximale du tube de plus grand diamètre, une extension longitudinale maximale, avec lesdites butées proximales de coulissement écartées ; et
      * l'extrémité distale de ladite unité télescopique présente, au-delà de l'extrémité distale du tube de plus grand diamètre, une extension longitudinale minimale.

La présente invention a pour objet un dispositif du type mentionné ci-dessus dans lequel l'introduction initiale de ladite unité de dilatation est facilitée, sans possibilité de recul d'un ou de plusieurs tubes.

A cette fin, selon l'invention, lesdites butées proximales de coulissement sont solidaires les unes des autres par l'intermédiaire de liaisons sécables.

Ainsi, le dispositif de dilatation conforme à la présente invention se présente sous la forme d'une unité, qui peut être introduite en une seule fois dans ledit canal corporel et lesdites butées proximales de coulissement sont successivement séparées les unes des autres au fur et à mesure de la poussée des tubes de diamètre croissant en direction de l'extrémité distale. Une telle unité peut être réalisée avec un faible diamètre, du fait qu'il est possible d'optimiser les jeux entre les tubes. On notera que l'introduction du dispositif de dilatation selon l'invention dans ledit canal corporel peut être ou non guidée par un fil-guide préalablement mis en place dans ledit canal.

Chaque butée proximale de coulissement peut être formée par un manchon tubulaire emboîté à l'extrémité proximale du tube correspondant, et donc traversé par les extrémités proximales des tubes de plus petit diamètre que celui-ci. Il peut être avantageux que le diamètre extérieur d'un tel manchon tubulaire soit d'autant plus grand que le diamètre du tube correspondant est plus grand.

Le nombre des tubes constituant le dispositif de dilatation conforme à la présente invention dépend du diamètre dudit canal, de la capacité de celui-ci à se dilater, de l'amplitude de la dilatation recherchée, etc ...

Dans le mode de réalisation le plus simple, ladite unité télescopique de dilatation est constituée de deux tubes emboîtés l'un dans l'autre, à savoir un tube intérieur de plus petit diamètre et un tube extérieur de plus grand diamètre.

Cependant, il est généralement nécessaire que ladite unité télescopique de dilatation soit constituée d'au moins trois tubes emboîtés les uns dans les autres, à savoir un tube intérieur de plus petit diamètre, un tube extérieur de plus grand diamètre et au moins un tube intermédiaire de diamètre intermédiaire. Ladite unité télescopique peut alors de plus prendre au moins une position de dilatation intermédiaire, entre ladite position d'introduction et ladite position de dilatation maximale, cette position de dilatation intermédiaire étant telle que l'extrémité proximale de ladite unité télescopique présente, au-delà de l'extrémité proximale du tube de plus grand diamètre, une extension longitudinale intermédiaire avec certaines desdites butées proximales de coulissement en contact, d'autres de ces butées étant écartées. Le nombre de tubes intermédiaires est par exemple égal à deux, de sorte que ladite unité télescopique de dilatation est alors constituée de quatre tubes.

Avantageusement, les tubes de ladite unité de dilatation portent des butées de coulissement supplémentaires pour définir ladite position de dilatation maximale, ainsi que chaque éventuelle position de dilatation intermédiaire. Ainsi, la course relative d'un tube par rapport au tube immédiatement adjacent est limitée par la distance séparant les deux butées de coulissement supplémentaires desdits tubes. De telles butées de coulissement supplémentaires peuvent être formées par des épaulements prévus sur lesdits tubes.

Le dispositif de dilatation conforme à la présente invention peut être réalisé en une matière plastique souple, par exemple un polyamide.

Les figures du dessin annexé feront bien comprendre comment l'invention peut être réalisée. Sur ces figures, des références identiques désignent des éléments semblables.

La figure 1 illustre en coupe longitudinale schématique, un premier mode de réalisation du dispositif de dilatation conforme à la présente invention en position d'introduction.

La figure 2 illustre, également en coupe longitudinale schématique, le dispositif de dilatation de la figure 1 en position de dilatation maximale.

La figure 3 montre, en coupe longitudinale, l'ensemble des butées proximales du dispositif de dilatation des figures 1 et 2.

Les figures 4, 5 et 6 illustrent, en coupe longitudinale schématique, un second mode de réalisation du dispositif de dilatation conforme à la présente invention, respectivement en position d'introduction, dans une position intermédiaire et en position de dilatation maximale.

La figure 7 montre, en coupe longitudinale, l'ensemble des butées proximales du dispositif de dilatation des figures 4, 5 et 6.

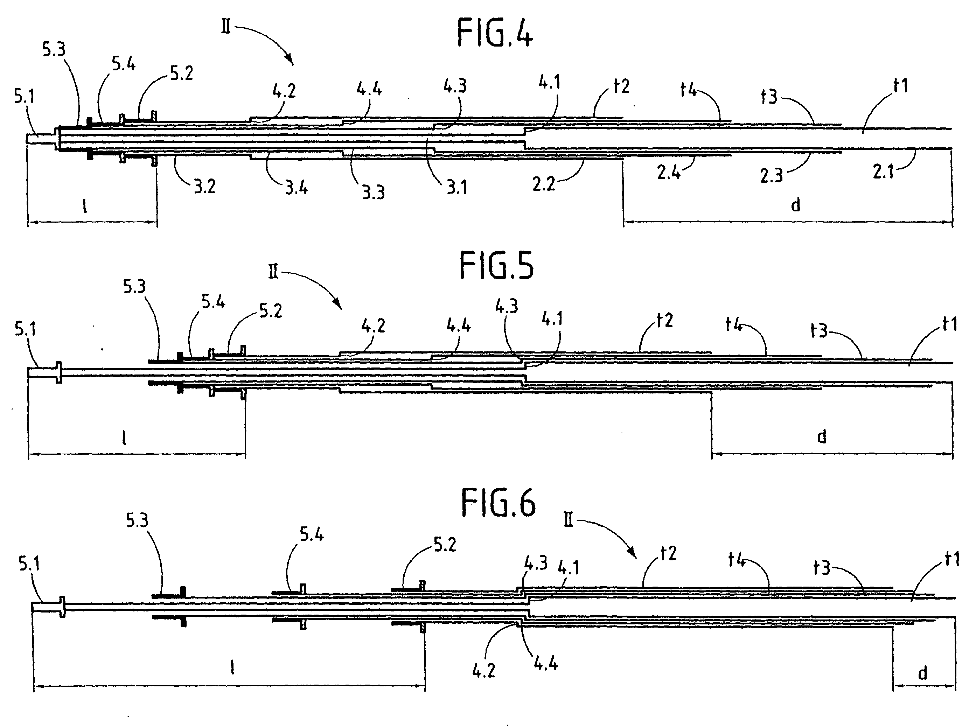

La figuré 8 est une coupe longitudinale d'une réalisation pratique du dispositif de dilatation des figures 4, 5 et 6.

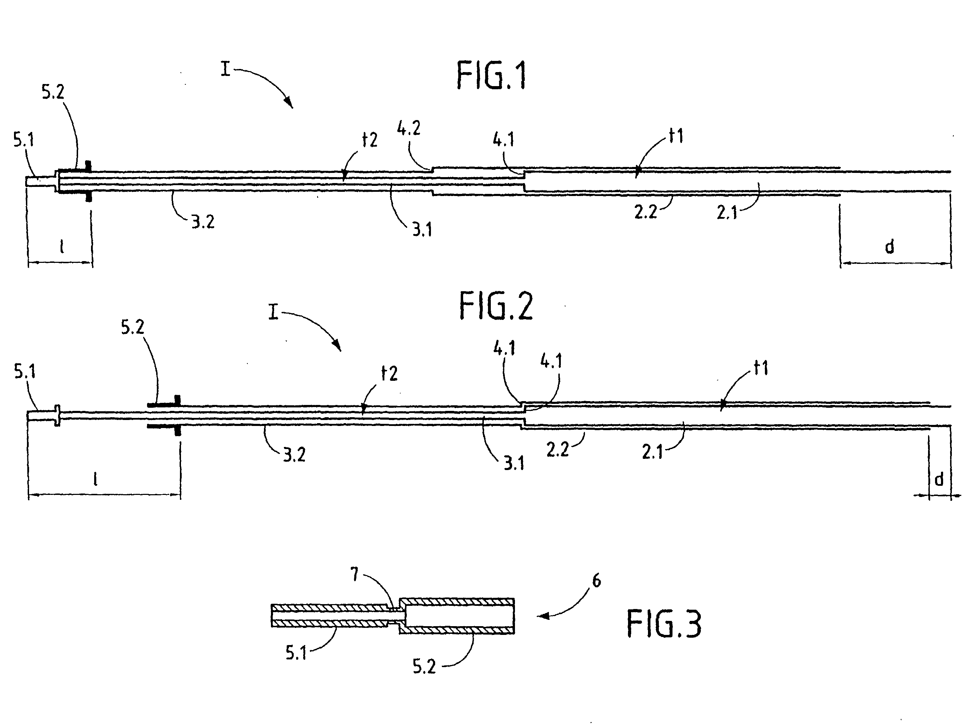

Le mode de réalisation I du dispositif de dilatation d'un canal corporel, conforme à la présente invention et montré sur les figures 1 et 2, comporte deux tubes t1 et t2 de diamètres différents susceptibles de coulisser l'un dans l'autre et formant une unité télescopique de dilatation.

Le tube intérieur t1, de plus petit diamètre, est plus long que le tube extérieur t2, de plus grand diamètre, de sorte que les extrémités proximale et distale dudit tube intérieur t1 sont en saillie, par rapport aux extrémités proximale et distale du tube extérieur t2.

Chacun desdits tubes t1 et t2 est formé par une partie distale 2.1 ou 2.2 raccordée à une partie proximale de plus petit diamètre 3.1 ou 3.2 par un épaulement 4.1 ou 4.2. De plus, l'extrémité proximale de chaque tube t1 et t2 est pourvue d'une butée proximale de coulissement 5.1 ou 5.2.

Comme le montre la figure 1, le dispositif de dilatation I peut prendre une première position, qui est celle de l'introduction dans un canal corporel et dans laquelle :
- l'extrémité proximale du dispositif I présente, au-delà de l'extrémité proximale du tube extérieur t2, une extension longitudinale l minimale, les butées proximales 5.1 et 5.2 étant au contact l'une de l'autre ;
- l'extrémité distale du dispositif I présente, au-delà de l'extrémité distale du tube extérieur t2, une extension longitudinale d maximale ; et
- les épaulements 4.1 et 4.2 sont écartés au maximum l'un de l'autre.

De plus, le dispositif de dilatation I peut prendre une seconde position, qui est représentée sur la figure 2 et dans laquelle :
- l'extension longitudinale proximale l est maximale, les butées 5.1 et 5.2 étant écartées l'une de l'autre ;
- l'extension longitudinale distale d est minimale ; et
- les épaulements 4.1 et 4.2 sont au contact l'un de l'autre.

Cette seconde position correspond à la dilatation maximale dudit canal corporel.

Comme cela est illustré par la figure 3, en position d'introduction (figure 1), les deux butées proximales 5.1 et 5.2 sont solidaires l'une de l'autre pour former un ensemble d'une seule pièce 6. Chaque butée 5.1 et 5.2 est formée par un manchon dans lequel est introduite et solidarisée l'extrémité proximale des parties 3.1 ou 3.2 des tubes t1 et t2 (non représentées sur la figure 3). Le manchon le plus proximal 5.1, associé au tube interne t1 de plus petit diamètre, présente un diamètre plus petit que le manchon 5.2, associé au tube extérieur t2. Une zone de rupture préétablie 7 relie, dans la pièce 6, les manchons 5.1 et 5.2.

Aussi, le dispositif de dilatation I étant dans son état d'introduction représenté sur la figure 1, il peut être introduit d'une seule pièce dans un canal corporel (non représenté), son extrémité distale étant essentiellement formée par la partie distale du tube intérieur de plus petit diamètre t1 en saillie par rapport à l'extrémité distale du tube extérieur de plus grand diamètre t2.

Pour dilater ledit canal, on casse alors la partie 7 de la pièce 6 et on pousse la butée 5.2 pour faire coulisser le tube extérieur t2 sur le tube intérieur t1, de sorte que l'extrémité distale de celui-ci est recouverte, au moins en partie, par l'extrémité distale du tube extérieur t2 de plus grand diamètre (voir la figure 2). Ainsi, ledit canal corporel est forcé à se dilater jusqu'à prendre le diamètre du tube extérieur t2.

Dans le mode de réalisation II des figures 4, 5 et 6 du dispositif de dilatation conforme à la présente invention, on retrouve les tubes intérieur et extérieur t1 et t2, avec leurs parties distales 2.1 et 2.2, leurs parties proximales 3.1 et 3.2, leurs épaulements 4.1 et 4.2 et leurs butées proximales 5.1 et 5.2. Cependant, dans ce mode de réalisation II, le dispositif de dilatation comporte deux tubes intermédiaires t3 et t4, disposés entre lesdits tubes intérieur et extérieur t1 et t2. Chacun desdits tubes intermédiaires t3 et t4 est formé par une partie distale 2.3 ou 2.4 raccordée à une partie proximale de plus petit diamètre 3.3 ou 3.4 par un épaulement 4.3 ou 4.4.

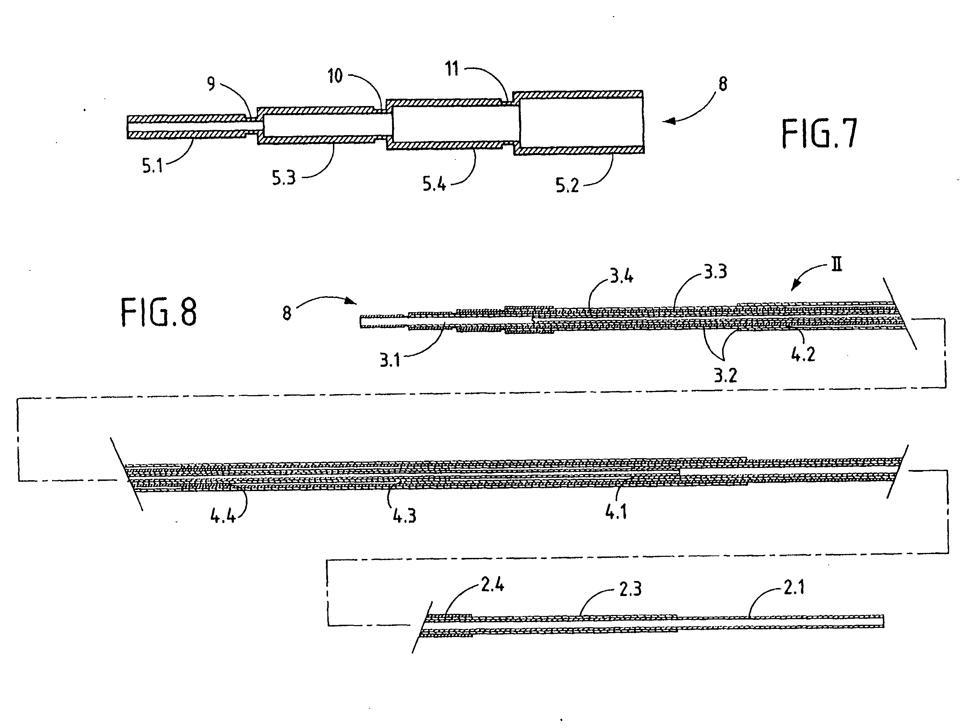

De plus, l'extrémité proximale de chaque tube intermédiaire t3 ou t4 est pourvue d'une butée proximale de coulissement 5.3 ou 5.4. Comme le montre la figure 7, les butées 5.1, 5.2, 5.3 et 5.4 sont constituées de manchons susceptibles d'être emboîtés et solidarisés des extrémités proximales desdites parties proximales 3.1, 3.2, 3.3 et 3.4 et forment une seule pièce 8, pourvue des zones de rupture préétablies 9, 10, 11 entre lesdits manchons. Ceux-ci peuvent présenter un diamètre d'autant plus grand que le tube auquel ils sont associés est plus grand.

Les tubes t1, t2, t3 et t4 sont emboîtés l'un dans l'autre pour former l'unité télescopique de dilatation II dans laquelle :
- la longueur d'un tube est d'autant plus grande que son diamètre est plus petit ;
- l'extrémité distale d'un tube est en saillie par rapport à l'extrémité distale du tube de diamètre immédiatement supérieur ;
- l'extrémité proximale d'un tube est en saillie par rapport à l'extrémité proximale du tube de diamètre immédiatement supérieur ; et
- l'extrémité proximale de chaque tube est pourvue d'une butée proximale de coulissement 5.1, 5.2, 5.3 ou 5.4.

Ainsi, le dispositif de dilatation II peut prendre (voir la figure 4) une position d'introduction dans le canal naturel, pour laquelle :
- l'extrémité proximale de ladite unité télescopique présente, au-delà de l'extrémité proximale du tube de plus grand diamètre t2, une extension longitudinale I minimale, avec lesdites butées proximales de coulissement 5.1, 5.2, 5.3 et 5.4 deux à deux en contact ;
- l'extrémité distale de ladite unité télescopique présente, au-delà de l'extrémité distale du tube de plus grand diamètre, une extension longitudinale d maximale ; et
- les épaulements 4.1, 4.2, 4.3 et 4.4 sont deux à deux écartés les uns des autres.

Le dispositif de dilatation II peut également prendre (voir la figure 6) une position de dilatation maximale dudit canal naturel, pour laquelle :
- l'extrémité proximale de ladite unité télescopique présente, au-delà de l'extrémité proximale du tube de plus grand diamètre, une extension longitudinale l maximale, avec lesdites butées proximales de coulissement deux à deux écartées ;
- l'extrémité distale de ladite unité télescopique présente, au-delà de l'extrémité distale du tube de plus grand diamètre, une extension longitudinale d minimale ; et
- les épaulements 4.1, 4.2, 4.3 et 4.4 sont au contact les uns des autres.

Par ailleurs, le dispositif de dilatation II peut prendre des positions intermédiaires, dont l'une est représentée par la figure 5. Dans cette position intermédiaire, les butées 5.3, 5.4 et 5.5 restent solidaires les unes des autres, mais ont été séparées de la butée 5.1. On conçoit aisément qu'une autre position intermédiaire (non représentée) correspond à la séparation de l'ensemble des butées 5.2 et 5.4 de la butée 5.3, elle-même séparée de la butée 5.1.

Le dispositif de dilatation II est introduit d'une seule pièce dans le canal corporel à dilater dans son état d'introduction représenté sur la figure 4. Ensuite, pour dilater ledit canal, on casse la partie 9 de la pièce 8, puis on pousse l'ensemble des tubes t2, t3, t4 jusqu'à ce que l'épaulement 4.3 vienne en butée contre l'épaulement 4.1 (voir la figure 5). Pour poursuivre la dilatation, on peut casser successivement les parties 10 et 11 de la pièce 8 et pousser d'abord les tubes t2 et t4 ensemble, puis le tube t2 seul jusqu'à obtenir la position de dilatation maximale de la figure 6, par coopération des épaulements 4.1 à 4.4. Bien entendu, éventuellement, une dilatation --non maximale-- peut être obtenue en s'abstenant de rompre l'une des parties 9, 10 ou 11 de la pièce 8.

Dans un mode de réalisation avantageux, les tubes t1, t2, t3 et t4 sont réalisés en un polyamide.

## Revendications

1. Dispositif (I, II) pour la dilatation d'un canal corporel comportant des tubes (t1, t2, t3, t4) de diamètres différents susceptibles de coulisser l'un dans l'autre, dispositif dans lequel :
- lesdits tubes sont emboîtés l'un dans l'autre pour former une unité télescopique de dilatation dans laquelle :
. la longueur d'un tube est d'autant plus grande que le diamètre de celui-ci est plus petit ;
. l'extrémité distale d'un tube est en saillie par rapport à l'extrémité distale du tube de diamètre immédiatement supérieur ;
. l'extrémité proximale d'un tube est en saillie par rapport à l'extrémité proximale du tube de diamètre immédiatement supérieur ; et
. l'extrémité proximale de chaque tube est pourvue d'une butée proximale de coulissement (5.1, 5.2, 5.3, 5.4) ; et
- ladite unité télescopique peut prendre au moins :
. une position d'introduction dans le canal naturel, pour laquelle :
* l'extrémité proximale de ladite unité télescopique présente, au-delà de l'extrémité proximale du tube de plus grand diamètre, une extension longitudinale (I) minimale, avec lesdites butées proximales de coulissement (5.1, 5.2, 5.3, 5.4) en contact ; et
* l'extrémité distale de ladite unité télescopique présente, au-delà de l'extrémité distale du tube de plus grand diamètre, une extension longitudinale (d) maximale ; et
. une position de dilatation maximale dudit canal naturel, pour laquelle :
* l'extrémité proximale de ladite unité télescopique présente, au-delà de l'extrémité proximale du tube de plus grand diamètre, une extension longitudinale (I) maximale, avec lesdites butées proximales de coulissement écartées (5.1, 5.2, 5.3, 5.4) ; et
* l'extrémité distale de ladite unité télescopique présente, au-delà de l'extrémité distale du tube de plus grand diamètre, une extension longitudinale (d) minimale.
**caractérisé en ce que** lesdites butées proximales de coulissement (5.1 à 5.4) sont solidaires les unes des autres par l'intermédiaire de liaisons sécables (7, 9, 10, 11).

2. Dispositif de dilatation selon la revendication 1,
**caractérisé en ce que** chaque butée proximale de coulissement (5.1 à 5.4) est formée par un manchon tubulaire emboîté à l'extrémité proximale du tube correspondant.

3. Dispositif de dilatation selon la revendication 2,
**caractérisé en ce que** le diamètre extérieur d'un tel manchon (5.1 à 5.4) est d'autant plus grand que le diamètre du tube correspondant (t1 à t4) est plus grand.

4. Dispositif de dilatation selon l'une quelconque des revendications 1 à 3,
**caractérisé en ce que** ladite unité télescopique de dilatation (I) est constituée de deux tubes (t1, t2) emboîtés l'un dans l'autre, à savoir un tube intérieur (t1) de plus petit diamètre et un tube extérieur (t2) de plus grand diamètre.

5. Dispositif de dilatation selon l'une quelconque des revendications 1 à 3,
**caractérisé en ce que** ladite unité télescopique de dilatation (II) est constituée d'au moins trois tubes (t1, t2, t3, t4) emboîtés les uns dans les autres, à savoir un tube intérieur de plus petit diamètre (t1), un tube extérieur de plus grand diamètre (t2) et au moins un tube intermédiaire de diamètre intermédiaire (t3, t4), de sorte que ladite unité télescopique peut de plus prendre au moins une position de dilatation intermédiaire, entre ladite position d'introduction et ladite position de dilatation maximale, cette position de dilatation intermédiaire étant telle que l'extrémité proximale de ladite unité télescopique présente, au-delà de l'extrémité proximale du tube de plus grand diamètre, une extension longitudinale intermédiaire avec certaines desdites butées proximales de coulissement en contact, d'autres de ces butées étant écartées.

6. Dispositif de dilatation selon l'une des revendications 1 à 5,
**caractérisé en ce que** lesdits tubes (t1 à t4) portent des butées de coulissement supplémentaires (4.1 à 4.4) pour définir ladite position de dilatation maximale.

7. Dispositif de dilatation selon les revendications 5 et 6,
**caractérisé en ce que** lesdites butées de coulissement supplémentaires (4.1 à 4.4) définissent de plus chaque position de dilatation intermédiaire.

8. Dispositif de dilatation selon l'une des revendications 2 ou 3,
**caractérisé en ce que** lesdites butées de coulissement supplémentaires (4.1 à 4.4) sont formées par des épaulements prévus sur lesdits tubes.

9. Dispositif de dilatation selon l'une des revendications 1 à 8,
**caractérisé en ce que** lesdits tubes (t1 à t4) sont réalisés en une matière plastique souple.

## Patentansprüche

1. Vorrichtung (I, II) zur Erweiterung eines Körperkanals, die Röhren (t1, t2, t3, t4) von unterschiedlichem Durchmesser umfasst, welche ineinander gleiten können, eine Vorrichtung, bei der:
- diese Röhren ineinandergefügt sind, um eine teleskopische Einheit zur Erweiterung zu bilden, bei der:
· die Länge einer Röhre um so größer ist, je geringer deren Durchmesser ist;
· das distale Ende einer Röhre, bezogen auf das distale Ende der Röhre mit dem nächstgrößeren Durchmesser, vorsteht;
· das proximale Ende einer Röhre, bezogen auf das proximale Ende der Röhre mit dem nächstgrößeren Durchmesser, vorsteht; und
· das proximale Ende jeder Röhre mit einem proximalen Gleitanschlag (5.1, 5.2, 5.3, 5.4) versehen ist; und
- die teleskopische Einheit wenigstens:
· eine Position der Einführung in den natürlichen Kanal einnehmen kann, bei der:
* das proximale Ende dieser teleskopischen Einheit jenseits des proximalen Endes der Röhre mit dem größten Durchmesser eine minimale Längsausdehnung (1) aufweist, wobei die proximalen Gleitanschläge (5.1, 5.2, 5.3, 5.4) in Kontakt sind; und
* das distale Ende dieser teleskopischen Einheit jenseits des distalen Endes der Röhre mit dem größten Durchmesser eine maximale Längsausdehnung (d) aufweist; und
· eine Position der maximalen Erweiterung des natürlichen Kanals einnehmen kann, bei der:
* das proximale Ende dieser teleskopischen Einheit jenseits des proximalen Endes der Röhre mit dem größten Durchmesser eine maximale Längsausdehnung (1) aufweist, wobei die proximalen Gleitanschläge (5.1, 5.2, 5.3, 5.4) beabstandet sind; und
* das distale Ende dieser teleskopischen Einheit jenseits des distalen Endes der Röhre mit dem größten Durchmesser eine minimale Längsausdehnung (d) aufweist;
**dadurch gekennzeichnet, dass** die proximalen Gleitanschläge (5.1 bis 5.4) durch trennbare Verbindungen (7, 9, 10, 11) miteinander verbunden sind:

2. Erweiterungsvorrichtung nach Anspruch 1, **dadurch gekennzeichnet, dass** jeder proximale Gleitanschlag (5.1 bis 5.4) von einer röhrenförmigen Muffe gebildet wird, die über das proximale Ende der entsprechenden Röhre geschoben ist.

3. Erweiterungsvorrichtung nach Anspruch 2, **dadurch gekennzeichnet, dass** der Außendurchmesser einer solchen Muffe (5.1 bis 5.4) um so größer ist, je größer der Durchmesser der entsprechenden Röhre (t1 bis t4) ist.

4. Erweiterungsvorrichtung nach einem der Ansprüche 1 bis 3, **dadurch gekennzeichnet, dass** die teleskopische Erweiterungseinheit (I) aus zwei ineinandergefügten Röhren (t1, t2) besteht, das heißt einer inneren Röhre (t1) mit einem kleineren Durchmesser und einer äußeren Röhre (t2) mit einem größeren Durchmesser.

5. Erweiterungsvorrichtung nach einem der Ansprüche 1 bis 3, **dadurch gekennzeichnet, dass** die teleskopische Erweiterungseinheit (II) aus wenigstens drei ineinandergefügten Röhren (t1, t2, t3, t4) besteht, das heißt einer inneren Röhre mit einem kleinsten Durchmesser (t1), einer äußeren Röhre mit einem größten Durchmesser (t2) und wenigstens einer dazwischen angeordneten Röhre mit einem dazwischenliegenden Durchmesser (t3, t4), so dass die teleskopische Einheit zusätzlich wenigstens eine Position der mittleren Erweiterung zwischen der Position der Einführung und der Position der maximalen Erweiterung einnehmen kann, wobei diese Position der mittleren Erweiterung derart ist, dass das proximale Ende der teleskopischen Einheit, jenseits des proximalen Endes der Röhre mit dem größten Durchmesser, eine mittlere Längsausdehnung aufweist, wobei einige der proximalen Gleitanschläge in Kontakt sind, andere dieser Anschläge beabstandet sind.

6. Erweiterungsvorrichtung nach einem der Ansprüche 1 bis 5, **dadurch gekennzeichnet, dass** die Röhren (t1 bis t4) zusätzliche Gleitanschläge (4.1 bis 4.4) tragen, um die Position der maximalen Erweiterung zu definieren.

7. Erweiterungsvorrichtung nach Anspruch 5 und 6, **dadurch gekennzeichnet, dass** die zusätzlichen Gleitanschläge (4.1 bis 4.4) außerdem jede Position der mittleren Erweiterung definieren.

8. Erweiterungsvorrichtung nach einem der Ansprüche 2 oder 3, **dadurch gekennzeichnet, dass** die zusätzlichen Gleitanschläge (4.1 bis 4.4) von auf den Röhren vorgesehenen Schultern gebildet werden.

9. Erweiterungsvorrichtung nach einem der Ansprüche 1 bis 8, **dadurch gekennzeichnet, dass** die Röhren (t1 bis t4) aus einem weichelastischen Kunststoff hergestellt sind.

## Claims

1. Device (I, II) for dilating a duct or vessel in the body, comprising tubes (t1, t2, t3, t4) of different diameters able to slide in one another, in which device:
- the said tubes are nested in one another to form a telescopic dilation unit in which:
• the smaller the diameter of a tube, the longer this tube is;
• the distal end of a tube projects with respect to the distal end of the tube of the next diameter up;
• the proximal end of a tube projects with respect to the proximal end of the tube of the next diameter up; and
• the proximal end of each tube is provided with a proximal sliding stop (5.1, 5.2, 5.3, 5.4); and
- the said telescopic unit can adopt at least:
• a position of insertion into the natural duct, for which:
* the proximal end of the said telescopic unit exhibits, beyond the proximal end of the largest-diameter tube, a minimum longitudinal extension (1), with the said proximal sliding stops (5.1, 5.2, 5.3, 5.4) in contact; and
* the distal end of the said telescopic unit exhibits, beyond the distal end of the largest-diameter tube, a maximum longitudinal extension (d); and
• a position of maximum dilation of the said natural duct, for which:
* the proximal end of the said telescopic unit exhibits, beyond the proximal end of the largest-diameter tube, a maximum longitudinal extension (1), with the said proximal sliding stops (5.1, 5.2, 5.3, 5.4) separated; and
* the distal end of the said telescopic unit exhibits, beyond the distal end of the largest-diameter tube, a minimum longitudinal extension (d),
**characterized in that** the said proximal sliding stops (5.1 to 5.4) are linked together by breakable connections (7, 9, 10, 11).

2. Dilation device according to Claim 1, **characterized in that** each proximal sliding stop (5.1 to 5.4) is formed of a tubular sleeve fitted onto the proximal end of the corresponding tube.

3. Dilation device according to Claim 2, **characterized in that** the outside diameter of such a sleeve (5.1 to 5.4) is larger the larger the diameter of the corresponding tube (t1 to t4).

4. Dilation device according to any one of Claims 1 to 3, **characterized in that** the said telescopic dilation unit (I) consists of two tubes (t1, t2) nested one inside the other, namely a smaller-diameter inner tube (t1) and a larger-diameter outer tube (t2).

5. Dilation device according to any one of Claims 1 to 3, **characterized in that** the said telescopic dilation unit (II) consists of at least three tubes (t1, t2, t3, t4) nested in one another, namely a smaller-diameter inner tube (t1), a larger-diameter outer tube (t2) and at least one intermediate-diameter intermediate tube (t3, t4), so that the said telescopic unit can in addition adopt at least one intermediate dilation position, somewhere between the said insertion position and the said maximum dilation position, this intermediate dilation position being such that the proximal end of the said telescopic unit exhibits, beyond the proximal end of the largest-diameter tube, an intermediate longitudinal extension with some of the said proximal sliding stops in contact, and others of these stops separated.

6. Dilation device according to one of Claims 1 to 5, **characterized in that** the said tubes (t1 to t4) bear additional sliding stops (4.1 to 4.4) to define the said maximum dilation position.

7. Dilation device according to Claims 5 and 6, **characterized in that** the said additional sliding stops (4.1 to 4.4) additionally define each intermediate dilation position.

8. Dilation device according to either of Claims 2 and 3, **characterized in that** the said additional sliding stops (4.1 to 4.4) are formed of shoulders provided on the said tubes.

9. Dilation device according to one of Claims 1 to 8, **characterized in that** the said tubes (t1 to t4) are made of flexible plastic.
